# EUROPEAN PATENT SPECIFICATION

(11) **EP 3 698 760 B1**
(45) Date of publication and mention of the grant of the patent: **25.03.2026**
(21) Application number: 19863968.4
(22) Date of filing: 17.01.2019
(51) Int. Cl.: A61F 2/40, A61F 2/30, A61F 2/34

(54) **SHOULDER JOINT PROSTHESIS**
SCHULTERGELENKPROTHESE
PROTHÈSE D'ARTICULATION D'ÉPAULE

(30) Priority: 27.12.2018 CN 201811614809
(43) Date of publication of application: 26.08.2020
(73) Proprietor: Beijing AK Medical Co., Ltd., 102200, Beijing (CN)
(72) Inventor: WANG, Caimei, Beijing 102200 (CN)
(74) Representative: Inchingalo, Simona
(86) International application number: PCT/CN2019/072100
(87) International publication number: WO 2020/133607

(56) References cited:
- EP-A1- 1 062 924
- WO-A1-2008/050091
- WO-A2-2008/080595
- CN-A- 104 490 497
- CN-A- 106 618 805
- CN-A- 107 582 219
- CN-A- 107 773 330
- CN-U- 206 424 180
- DE-A1- 2 829 676
- DE-U1- 202012 100 571
- US-A1- 2003 114 935
- US-A1- 2007 179 624
- US-A1- 2008 114 459
- US-A1- 2011 137 424

## Description

### Technical Field

The disclosure relates to a technical field of orthopedic medical instruments, and in particular to a shoulder joint prosthesis.

### Background

A shoulder joint consists of a glenoid of a scapula and a head of humerus. There is a great difference between two connected bony articular surfaces, and specifically, a glenoid fossa may receive 1/4-1/3 of an articular head only. A labrum articulare on a periphery of the glenoid fossa may deepen the glenoid. A joint capsule is thin and floppy, and is attached between a periphery of the glenoid and a humeral anatomical neck. Furthermore, a wall of the joint capsule further has a tendon of long head of biceps brachii wrapped by a synovial membrane therein. The shoulder joint may be reinforced through the tendon.

The shoulder joint is the most flexible ball and socket joint of the whole body and may bend, stretch, fold, unfold, rotate and implement circumduction. In addition, there is a great difference between areas of the articular head and the glenoid fossa, and the joint capsule is thin and floppy, a flexible exercising function of the shoulder joint is reflected. There are a large number of muscles around the shoulder joint, and these muscles are of great significance to maintain stability of the shoulder joint. However, there is few muscle forward and below the shoulder joint, and the joint capsule is the floppiest part; therefore the shoulder joint is a weak point having the worst stability of the joint. When falling back at an abducent position and an extorsion position of an upper limb, a person may land on a palm or an elbow, thereby easily causing anterior dislocation of the shoulder joint.

A total shoulder arthroplasty may be applicable to osteoarthritis. The osteoarthritis may include primary and secondary rheumatoid arthritis, traumatic osteoarthritis, rotator cuff traumatic arthropathy. Furthermore, the total shoulder arthroplasty may further be applicable to revision of an artificial shoulder joint, osteonecrosis, tumor, shoulder dysplasia, old infection and the like.

An artificial shoulder arthroplasty is defined to prepare an artificial joint prosthesis from metal, high-molecular polyethylene, ceramic and other materials in accordance with a form, a structure and a function of a human joint, and implant into the person through a surgical technology, to substitute a function of a sick joint and accordingly achieve an objective of relieving joint ache and recovering the function of the shoulder joint.

The artificial shoulder joint is an artificial organ of replacing the damaged shoulder joint. The artificial shoulder arthtoplasty was applied to clinic almost at the same time with an artificial hip arthroplasty and an artificial knee arthroplasty. However, the artificial shoulder arthroplasty may not be compared with the artificial hip arthroplasty and the artificial knee arthroplasty in the aspects of implementation quantity and long-term effect. It is mainly because the shoulder joint has a great range of motion, and a patient has high requirement to living quality, while a function rehabilitation level after joint reconstruction may depend on a condition of a periarticular soft tissue to a large extent.

The artificial shoulder joint occurs due to the need of proximal humeral fracture. Many scholars have proposed various types of artificial shoulders, mainly including Neerl type artificial shoulder developed by Neer in 1953. Subsequently, the Neerl type artificial shoulder was developed into Neerll type and Neerlll type artificial shoulders in accordance with a clinic test.

On this basis, the artificial shoulder joint has been constantly improved in accordance with different requirements, and a new artificial shoulder joint constantly occurs. Generally, the artificial shoulder may include an artificial half-shoulder joint and an artificial full-shoulder joint.

An artificial head of humerus is mainly applied to a case of serious impairment, such as comminuted fracture and necrosis of the head of humerus, a head of costa, a proximal humerus while a rotator cuff is normal and a glenoid articular surface is lightly damaged, for implantation of artificial half-shoulder arthroplasty. An artificial humeral prosthesis has been applied so far, with a maximum quantity.

As for the damaged glenoid articular surface, the glenoid surface may be replaced with a cylindrical polyethylene prosthesis or a disc-shaped polyethylene prosthesis when replacing the artificial head of humerus. It is called an artificial full-shoulder joint arthroplasty. The artificial full-shoulder joint may be applied to various joint diseases, such as osteoarthrosis, rheumatoid arthritis and bone tumor. A bone structure may be seriously damaged and may not be repaired easily due to the bone tumor of an upper end of the humerus, and the proximal humerus needs to be cut off if necessary, to implement the artificial shoulder joint arthroplasty.

As for the shoulder joint tumor, especially the bone tumor of the upper end of humerus, the bone structure, and even the rotator cuff may be seriously damaged and may not be repaired easily due to the tumor resection of the proximal humerus. A routine artificial shoulder joint is an unrestricted joint which implements traction through the human muscles and the soft tissues. Therefore, stability of the artificial shoulder joint may not be offered during motion, and dislocation may be caused in a severe case.

An example of a hip joint prosthesis according to the prior art is known from WO 2008080595 A2.

### Summary

The invention is set out in the appended set of claims.

The disclosure is intended to provide a shoulder joint prosthesis, to solve the problem that an unrestricted artificial shoulder joint in the device known to the inventors has poor stability during motion.

In order to achieve the abovementioned objective, a shoulder joint prosthesis in accordance with the disclosure may include an outer cup, a lining, a ball head and a humeral shank. The outer cup, the lining and the ball head are nested in sequence. wherein, the outer cup may include an outer cup body and a first limiting convex wall provided at an opening of the outer cup body, the outer cup body is provided with a first spherical inner cavity, the first limiting convex wall protrudes out of a center surface of the first spherical inner cavity, the center surface of the first spherical inner cavity is a plane passing a spherical center of the first spherical inner cavity and vertical to an axis of the outer cup, and an end surface of the outer cup body is lower than the center surface of the first spherical inner cavity. The lining may include a hemispherical housing body and a second limiting convex wall and a third limiting convex wall provided at an opening of the hemispherical housing body. The second limiting convex wall and the third limiting convex wall protrude out of a center surface of the hemispherical housing body. The center surface of the hemispherical housing body is a plane passing a spherical center of the hemispherical housing body and vertical to an axis of the lining. The hemispherical housing body is provided with a second spherical inner cavity for accommodating the ball head. The second limiting convex wall and the third limiting convex wall limit the ball head to leave the second spherical inner cavity. The hemispherical housing body is provided with a groove for avoiding the humeral shank. The groove is lower than the center surface of the hemispherical housing body, and the lining is mounted inside the first spherical inner cavity in a rotational motion. Wherein, the first limiting convex wall is positioned outside the second limiting convex wall, as to limit the lining to leave the first spherical inner cavity.

In some embodiments, a protrusion height of the second limiting convex wall is greater than that of the third limiting convex wall.

There are two third limiting convex walls provided oppositely, and the second limiting convex wall is positioned between the two third limiting convex walls. There are multiple grooves, and the multiple grooves include two first grooves between the two third limiting convex walls and the second limiting convex wall and a second groove configured between the two third limiting convex walls.

In some embodiments, the lining is an axisymmetric structure, and the second grooves and the second limiting convex wall are provided oppositely.

A position on an end surface of the outer cup body, corresponding to the second groove, is provided with a first section structure for avoiding the humeral shank.

A position on an end surface of the outer cup body, corresponding to one of the two first grooves, is provided with a second section structure for avoiding the humeral shank.

In some embodiments, the groove is an arc groove.

In some embodiments, a cavity wall of the first spherical inner cavity is provided with an annular groove. There are multiple annular grooves provided at intervals along an axial direction of the outer cup body.

The cavity wall of the first spherical inner cavity is provided with a clamping slot. The clamping slot extends along a peripheral direction of the cavity wall of the first spherical inner cavity, and an outer wall surface of the lining is provided with a clamping rib corresponding to the clamping slot.

In some embodiments, the shoulder joint prosthesis may include multiple fasteners. A bottom of the outer cup body is provided with multiple connecting holes, and the multiple connecting holes are counter bore structures.

In some embodiments, multiple included angles are formed between axes of the multiple connecting holes and a central axis of the outer cup body, degrees of the plurality of included angles are different.

With the adoption of the technical solution of the disclosure, the shoulder joint prosthesis may include the outer cup, the lining, the ball head and the humeral shank. The outer cup, the lining and the ball head are nested in sequence. In the disclosure, the outer cup may include the outer cup body and the first limiting convex wall provided at the opening of the outer cup body, the outer cup body has the first spherical inner cavity, the first limiting convex wall protrudes out of the center surface of the first spherical inner cavity, the center surface of the first spherical inner cavity is the plane passing the spherical center of the first spherical inner cavity and vertical to the axis of the outer cup, and the end surface of the outer cup body is lower than the center surface of the first spherical inner cavity. The first limiting convex wall protrudes out of the center surface of the first spherical inner cavity, in this way a part provided inside the first spherical inner cavity may be limited. The lining may include the hemispherical housing body and the second limiting convex wall and the third limiting convex wall provided at the opening of the hemispherical housing body. The second limiting convex wall and the third limiting convex wall protrude out of the center surface of the hemispherical housing body, and the center surface of the hemispherical housing body is the plane passing the spherical center of the hemispherical housing body and vertical to the axis of the lining. The hemispherical housing body has the second spherical inner cavity for accommodating the ball head, and the second limiting convex wall and the third limiting convex wall limit the ball head to leave the second spherical inner cavity. The hemispherical housing body is provided with the groove for avoiding the humeral shank. The groove is lower than the center surface of the hemispherical housing body. In the disclosure, the ball head is mounted inside the second spherical inner cavity of the lining first when assembling the outer cup, the lining and the ball head. The second limiting convex wall and the third limiting convex wall protrude out of the center surface of the hemispherical housing body, in this way the ball head inside the second spherical inner cavity may be limited. The lining and the ball head when assembled are mounted inside the first spherical inner cavity in a rotational motion. In the disclosure, the shoulder joint prosthesis may be mounted on a scapula of a patient. Under the situation that the lining is provided inside the outer cup, the first limiting convex wall is positioned outside the second limiting convex wall, as to limit the lining to leave the first spherical inner cavity. The ball head may move in the second spherical inner cavity rotatably, and the second limiting convex wall and the third limiting convex wall limit the ball head from to leave the second spherical inner cavity. The first spherical inner cavity may accommodate the lining, and the lining may not be deformed due to the first spherical inner cavity. In this way, the ball head is further ensured not to leave the second spherical inner cavity. Therefore, the ball head may rotate freely in the second spherical inner cavity, without leaving the lining. Accordingly, the ball head may be effectively prevented from dislocation on the premise that a freedom of motion of the ball head is ensured. In the disclosure, the outer cup, the lining and the ball head of the shoulder joint prosthesis are connected in a mode of nesting in sequence rather than implement traction through the human muscles and the soft tissues in the conventional art. Accordingly, the stability of the shoulder joint prosthesis is ensured during the motion.

### Brief Description of the Drawings

The accompanying drawings described herein are used to provide a further understanding of the disclosure, and constitute a part of the present application, and the exemplary embodiments of the disclosure and the description thereof are used to explain the disclosure, but do not constitute improper limitations to the disclosure. In the drawings:
Fig. 1 shows a stereo structure diagram of an embodiment in accordance with a shoulder joint prosthesis of the disclosure.
Fig. 2 shows a stereo structure diagram of a shoulder joint prosthesis in Fig. 1 from another angle.
Fig. 3 shows a structure diagram of a humeral shank of a shoulder joint prosthesis in Fig. 1 at two first grooves.
Fig. 4 shows a stereo structure diagram of a shoulder joint prosthesis in Fig. 1 after removal of a ball head and a humeral shank.
Fig. 5 shows a front view of an outer cup and a fastener of a shoulder joint prosthesis in Fig. 1.
Fig. 6 shows an A-A section view of an outer cup and a fastener in Fig. 5.
Fig. 7 shows a left view of an outer cup and a fastener in Fig. 5.
Fig. 8 shows a stereo structure diagram of an outer cup of a shoulder joint prosthesis in Fig. 1.
Fig. 9 shows a stereo structure diagram of an outer cup in Fig. 8 from another angle.
Fig. 10 shows a front view of an outer cup in Fig. 8.
Fig. 11 shows a B-B section view of an outer cup in Fig. 8.
Fig. 12 shows a left view of an outer cup in Fig. 8
Fig. 13 shows a front view of a lining of a shoulder joint prosthesis in Fig. 1.
Fig. 14 shows a C-C section view of a lining in Fig. 13.
Fig. 15 shows a top view of a lining of a shoulder joint prosthesis in Fig. 1.
Fig. 16 shows a left view of a lining in Fig. 13.
Fig. 17 shows a stereo structure diagram of a clamping slot of a shoulder joint prosthesis in Fig. 1.
Fig. 18 shows a section view when a clamping slot of a shoulder joint prosthesis in Fig. 1 cooperates with a clamping rib.
Fig. 19 shows an assembling process diagram of an outer cup and a lining of a shoulder joint prosthesis in Fig. 1.

Herein, the abovementioned drawings may include reference numbers below:
10: Outer cup; 11: Outer cup body; 111: First spherical inner cavity; 1111: Annular groove; 1112: Clamping slot; 112: First section structure; 113: Second section structure; 114: Connecting hole; 12: First limiting convex wall; 20: Ball head; 21: Humeral shank; 211: Connecting column; 30: Lining; 31: Hemispherical housing body; 32: Second limiting convex wall;; 33: Third limiting convex wall; 34: Second spherical inner cavity; 35: Groove; 351: First groove; 352: Second groove; 36: Clamping rib; 40: Fastener.

### Detailed Description of the Embodiments

The technical solutions in the embodiments of the disclosure will be clearly and completely described below in combination with the drawings in the embodiments of the disclosure. It is apparent that the described embodiments are not all embodiments but part of embodiments of the disclosure. The description of at least one exemplary embodiment below is illustrative only in fact, and will never be taken as any limitations to the disclosure and its application or use. All other embodiments obtained by those of ordinary skilled in the art on the basis of the embodiments in the disclosure without creative work shall fall within the scope of protection of the disclosure, which is solely defined by the appended claims.

It is to be noted that terms used here are to describe a specific embodiment only and not intended to limit the exemplary embodiment in accordance with the application. If they are used here, unless otherwise clearly specified in the context, a singular form should be intended to include a plural form. In addition, it is to be understood that when the term "include" and/or "comprise" is used in the description, it indicates that there is a feature, a step, an operation, a device, a component and/or their combinations.

Unless otherwise specifically stated, a relative arrangement of the part and the step, a number expression and a value clarified in these embodiments may not limit the scope of the disclosure. Meanwhile, it is to be understood that, in order to facilitate description, a size of each part shown in the figure is not drawn in accordance with an actual proportional relation. A technology, a method and a device known by those of ordinary skilled in the related art may not be discussed in detail. However, the technology, the method and the device should be deemed as one part of an authorization description where appropriate. In all examples shown and discussed here, any specific values should be explained to be exemplary merely rather than be restrictive. Therefore, other examples of the exemplary embodiments may have different values. It is to be noted that a similar reference number and a similar letter may represent similar terms in the figure below. Therefore, once defined in one figure, a certain item does not need to be further discussed in a subsequent figure.

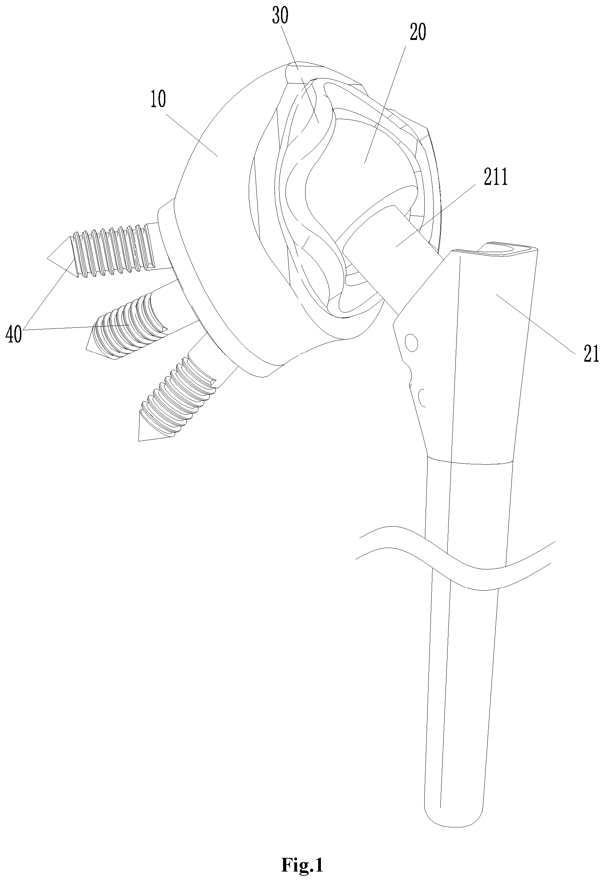

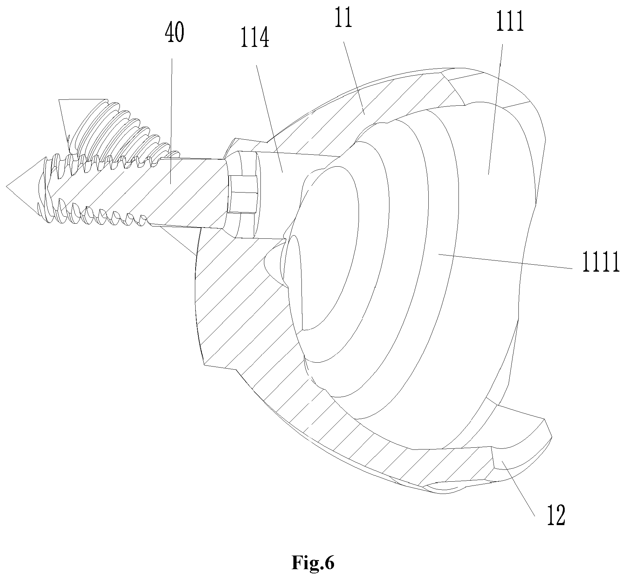

As shown in Fig. 1, Fig. 3 and Fig. 6, a shoulder joint prosthesis of the embodiment includes an outer cup 10, a lining 30, a ball head 20 and a humeral shank 21, and the outer cup 10, the lining 30 and the ball head 20 are nested in sequence. Wherein, the outer cup 10 includes an outer cup body 11 and a first limiting convex wall 12 provided at an opening of the outer cup body 11, the outer cup body 11 has a first spherical inner cavity 111, the first limiting convex wall 12 protrudes out of a center surface of the first spherical inner cavity 111, the center surface of the first spherical inner cavity 111 is a plane passing a spherical center of the first spherical inner cavity 111 and vertical to an axis of the outer cup 10, and an end surface of the outer cup body 11 is lower than the center surface of the first spherical inner cavity 111. The lining 30 includes a hemispherical housing body 31 and a second limiting convex wall 32 and a third limiting convex wall 33 provided at an opening of the hemispherical housing body 31. The second limiting convex wall 32 and the third limiting convex wall 33 protrude out of a center surface of the hemispherical housing body 31. The center surface of the hemispherical housing body 31 is a plane passing a spherical center of the hemispherical housing body 31 and vertical to an axis of the lining 30. The hemispherical housing body 31 has a second spherical inner cavity 34 for accommodating the ball head 20. The second limiting convex wall 32 and the third limiting convex wall 33 limit the ball head 20 to leave the second spherical inner cavity 34. The hemispherical housing body 31 is provided with a groove 35 for avoiding the humeral shank 21. The groove 35 is lower than the center surface of the hemispherical housing body 31, and the lining 30 is mounted inside the first spherical inner cavity 111 in a rotational motion. Wherein, under a situation that the lining 30 is provided inside the outer cup 10, the first limiting convex wall 12 is positioned outside the second limiting convex wall 32, as to limit the lining 30 to leave the first spherical inner cavity 111.

With the adoption of the technical solution of the embodiment, the shoulder joint prosthesis includes the outer cup 10, the lining 30, the ball head 20 and the humeral shank 21. The outer cup 10, the lining 30 and the ball head 20 are nested in sequence. In the embodiment, the ball head 20 is mounted inside the second spherical inner cavity 34 of the lining 30 first when assembling the outer cup 10, the lining 30 and the ball head 20. The second limiting convex wall 32 and the third limiting convex wall 33 protrude out of the center surface of the hemispherical housing body 31, thereby achieving limitation of the ball head 20 inside the second spherical inner cavity 34. The lining 30 and the ball head 20 when assembled are mounted inside the first spherical inner cavity 111 in the rotational motion. In the embodiment, the shoulder joint prosthesis may be mounted on a scapula of a patient. Under the situation that the lining 30 is provided inside the outer cup 10, the first limiting convex wall 12 is positioned outside the second limiting convex wall 32, as to limit the lining 30 to leave the first spherical inner cavity 111. The ball head may move in the second spherical inner cavity 34 rotatably, and the second limiting convex wall 32 and the third limiting convex wall 33 limit the ball head to leave the second spherical inner cavity 34. The first spherical inner cavity 111 accommodates the lining 30, and the lining 30 may not be deformed due to the first spherical inner cavity 111. In this way, the ball head 20 is further ensured not to leave the second spherical inner cavity 34. Therefore, the ball head 20 may rotate freely in the second spherical inner cavity 34, without leaving the lining 30. Accordingly, the ball head 20 may be effectively prevented from dislocation on the premise that a freedom of motion of the ball head 20 is ensured. In the embodiment, the outer cup 10, the lining 30 and the ball head 20 of the shoulder joint prosthesis are connected in a mode of nesting in sequence rather than implement traction through human muscles and soft tissues in the conventional art. Accordingly, stability of the shoulder joint prosthesis is ensured during motion.

It is to be noted that the axis of the outer cup 10 overlaps with the axis of the lining 30, and the axis of the outer cup 10 overlaps with the axis of the shoulder joint prosthesis as well.

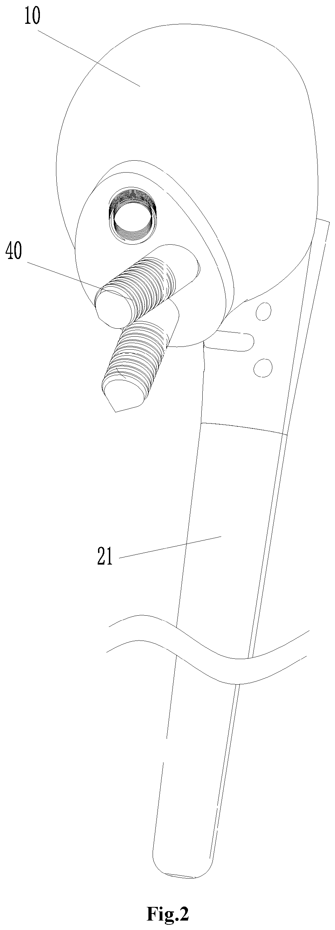

As shown in Figs. 1-4, in the embodiment, the humeral shank 21 may include a connecting column 211 connecting with the ball head 20, and the groove 35 on the hemispherical housing body 31 is configured to avoid the connecting column 211 of the humeral shank 21. The groove 35 may effectively avoid the connecting column 211 when the connecting column 211 rotates along the ball head 20. In this way, the humeral shank 21 may have a greater scope of motion and move more flexibly.

As shown in Figs. 1-4, in the embodiment, a protrusion height of the second limiting convex wall 32 is greater than that of the third limiting convex wall 33. Under the situation that the lining 30 is provided inside the outer cup 10, the second limiting convex wall 32 may cooperate with the first limiting convex wall 12, as to prevent the lining 30 from leaving the second spherical inner cavity 34. The protrusion height of the second limiting convex wall 32 is great, thereby more excellently ensuring a dislocation prevention effect. In addition, an arm folds forward with a great degree of motion and stretches backward with a small degree of motion when a shoulder joint of a person moves. Therefore, when the shoulder joint prosthesis is connected into a patient, compared with the second limiting convex wall 32, the third limiting convex wall 33 limits the motion of the humeral shank 21, with a weak limitation effect. In this way, the effect of limiting the arm of the person during motion may be achieved through the protrusion height of the second limiting convex wall 32 greater than that of the third limiting convex wall 33.

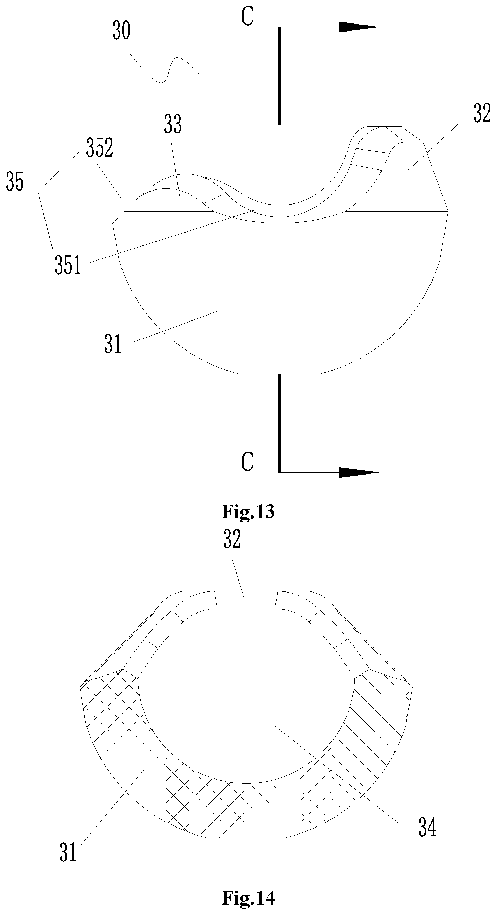

As shown in Fig. 1 and Fig. 13, in the embodiment, there are two third limiting convex walls 33 provided oppositely, and the second limiting convex wall 32 is positioned between the two third limiting convex walls 33. There are three grooves 35, and the three grooves 35 include two first grooves 351 between the two third limiting convex walls 33 and the second limiting convex wall 32 and a second groove 352 between the two third limiting convex walls 33. When the connecting column 211 rotates along a circumferential direction of the ball head 20, the two first grooves 351 and the two second grooves 352 may avoid rotation of the connecting column 211. Accordingly, the groove 35 may provide a sufficient space of motion when the humeral shank 21 rotates. As for the humeral shank 21, it needs to move in a front area, an upper area and a lower area generally. When the shoulder joint prosthesis is connected into the patient, the second limiting convex wall 32 and the first limiting convex wall 12 need to be positioned at a rear side of the humeral shank 21. In this way, the abovementioned three grooves 35 may correspond to the front area, the upper area and the lower area respectively, and accordingly the humeral shank 21 may have a great scope of motion in the abovementioned areas. As for the abovementioned scope of motion, the rear side of the humeral shank 21 may need a small scope of motion, in this way the second limiting convex wall 32 and the first limiting convex wall 12 positioned at the rear side may provide limitation sufficiently.

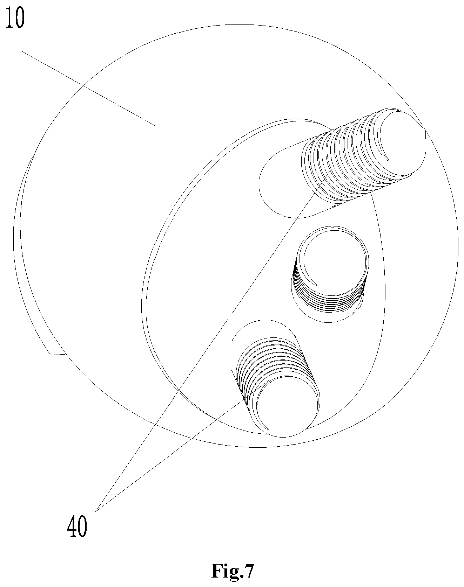

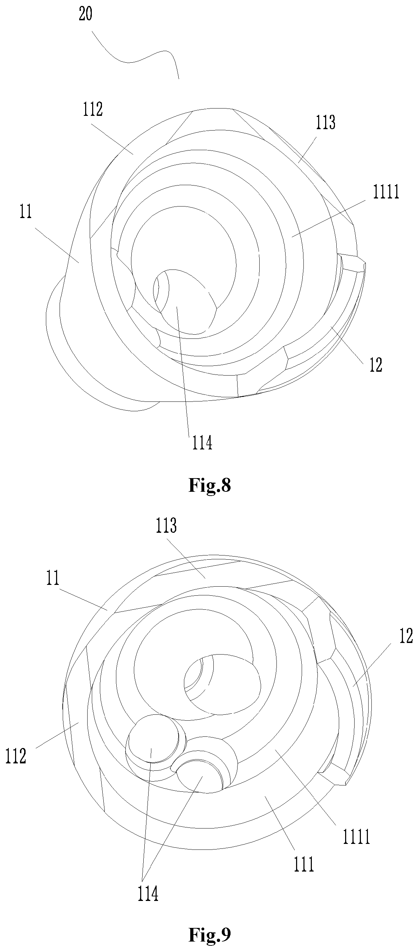

As shown in Figs. 1-13, in the embodiment, the lining 30 is an axisymmetric structure, in this way processing may be facilitated compared with an irregular appearance having a non-axisymmetric lining. The second groove 352 and the second limiting convex wall 32 are provided oppositely, thereby facilitating mounting of the lining 30.

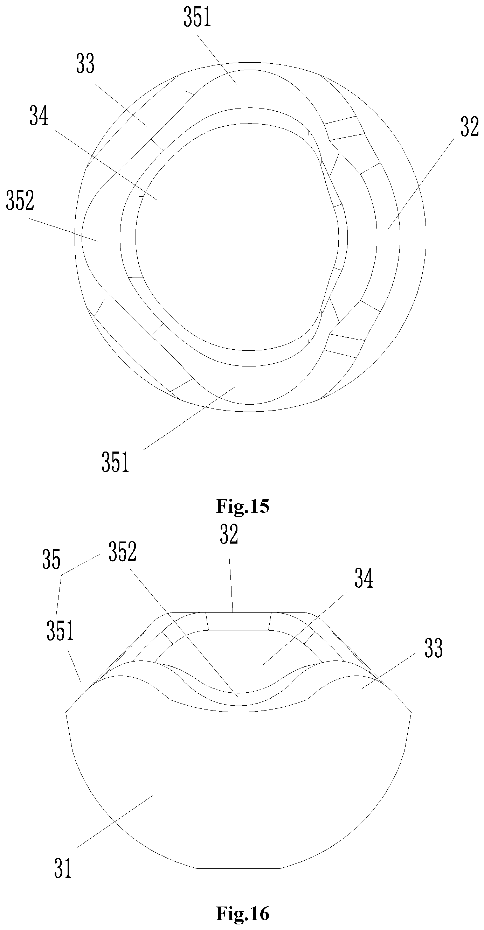

As shown in Fig. 1, Fig. 8 and Fig. 16, in the embodiment, the humeral shank 21 may further include a humeral shank body connecting with the connecting column 211 and a bent neck part. A certain angle may be formed by the bent neck part and the humeral shank body. A position on the end surface of the outer cup body 11, corresponding to the second groove 352, is provided with a first section structure 112 for avoiding the humeral shank 21. In this way, when the humeral shank 21 rotates around the ball head 20, the first section structure 112 may avoid the bent neck part, and accordingly the humeral shank 21 may rotate smoothly.

As shown in Fig. 1, Fig. 8 and Fig. 16, in the embodiment, a position on the end surface of the outer cup body 11, corresponding to one of the two first grooves 351, is provided with a second section structure 113 for avoiding the humeral shank 21. In this way, when the humeral shank 21 rotates around the ball head 20, the second section structure 113 may avoid the bent neck part, and accordingly the humeral shank 21 may rotate smoothly.

As shown in Fig. 1, Fig. 13 and Fig. 16, in the embodiment, when the humeral shank 21 rotates around the ball head 20, the groove 35 is an arc groove in order to make the connecting column 211 achieve stable transition when passing the groove 35. A structure of the arc groove may ensure that the humeral shank 21 has a maximum possible degree of freedom when the humeral shank 21 rotates.

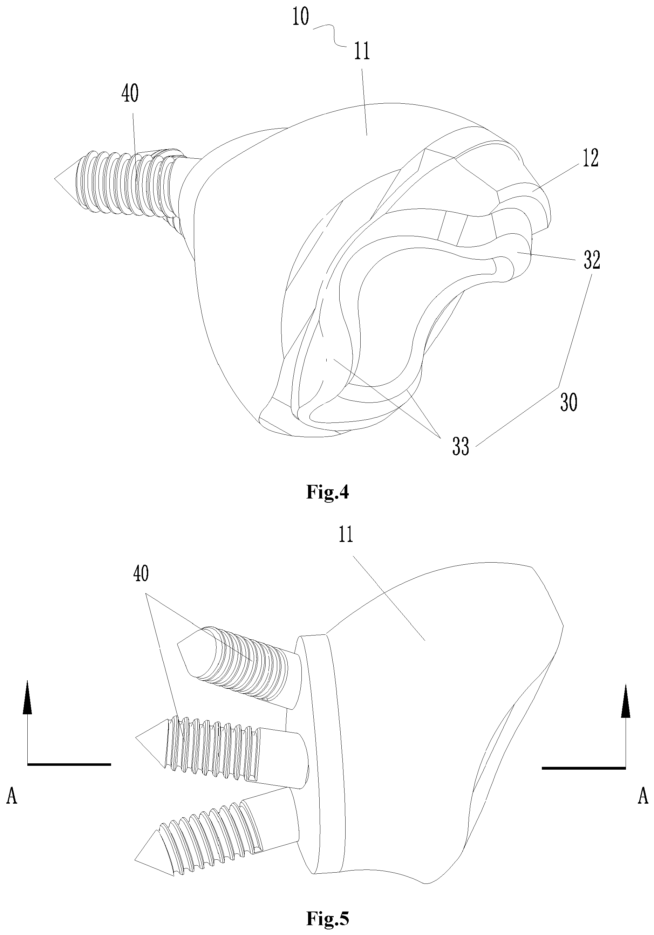

As shown in Figs. 4-6 and Fig. 11, a cavity wall of the first spherical inner cavity 111 is provided with an annular groove 1111. There are two annular grooves 1111 provided along an axial direction of the outer cup body 11 at intervals. In the embodiment, the lining 30 is mounted into the outer cup body 11, and there is a gap between an outer wall surface of the lining 30 and the first spherical inner cavity 111. Therefore, bone cement needs to be added to the first spherical inner cavity 111, for auxiliary fixation. An arrangement of the annular groove 1111 facilitates addition of the bone cement into the first spherical inner cavity 111. The annular groove 111 may effectively accommodate the bone cement. The bone cement is added to the annular groove 1111, thereby making the first spherical inner cavity 111 adhered to the outer wall surface of the lining 30. In the embodiments not shown by other figures, a quantity of the annular grooves may be 1, 3 and above.

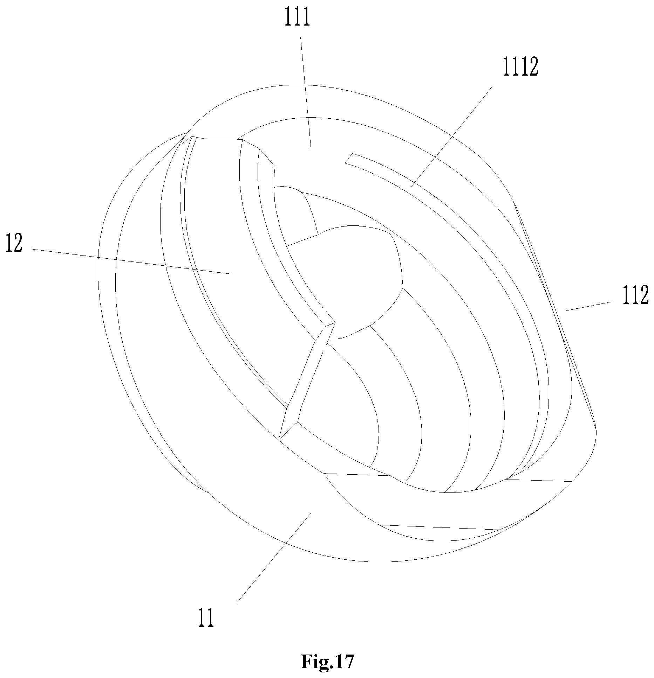

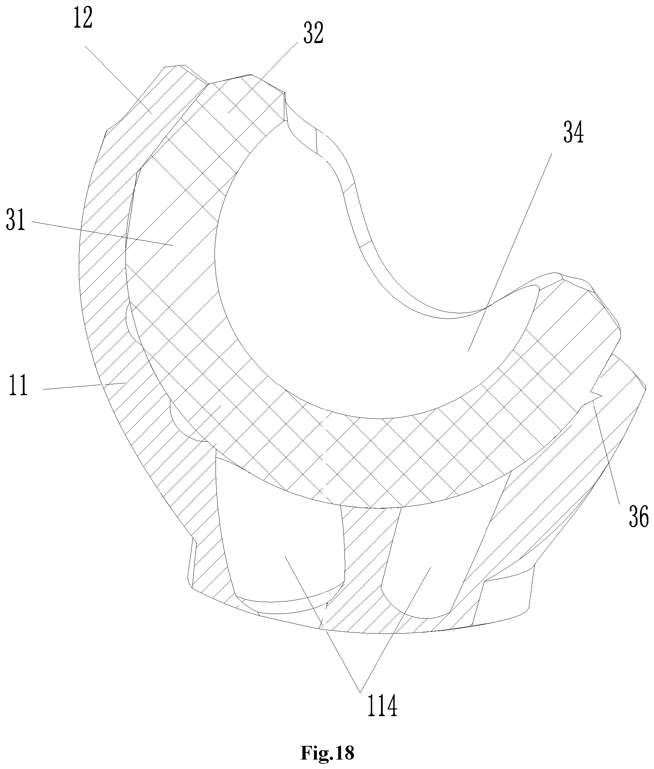

As shown in Fig. 17 and Fig. 18, the cavity wall of the first spherical inner cavity 111 is further provided with a clamping slot 1112. The clamping slot 1112 extends along a circumferential direction of the cavity wall of the first spherical inner cavity 111. The outer wall surface of the lining 30 is provided with a clamping rib 36 corresponding to the clamping slot 1112. The clamping rib 36 includes a slope portion and a plane portion connecting with the slope portion. The slope portion is provided obliquely relative to the axis of the lining 30, and the plane portion is vertical to the axis of the lining 30. In this way, the clamping rib 36 may be easily and smoothly clamped into the clamping slot 1112, with a good effect of preventing dislocation. In the embodiment, the lining 30 may be extruded when mounted into the first spherical inner cavity 111. In this way, the clamping rib 36 may enter into the clamping slot 1112, and the plane portion may be tightly clamped in the clamping slot 1112. Accordingly, the clamping slot 1112 cooperates with the clamping rib 36, thereby making the lining 30 not leave the first spherical inner cavity 111 easily.

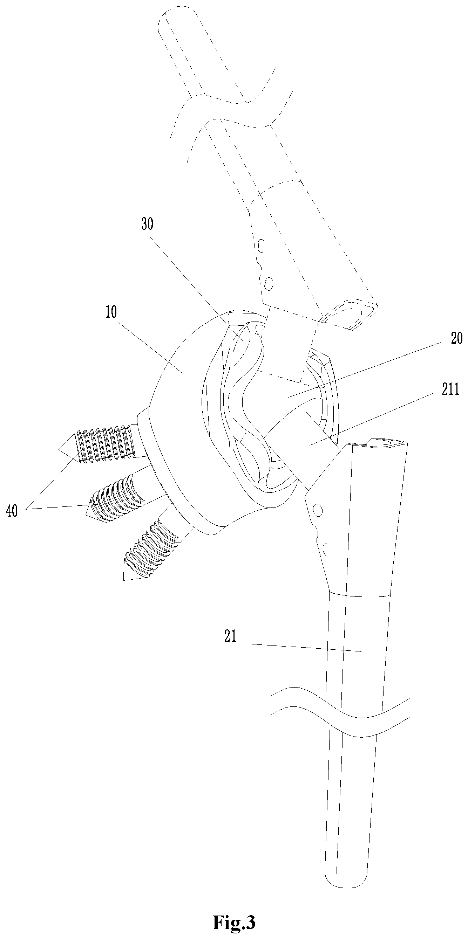

As shown in Fig. 3, Fig. 6 and Fig. 7, the shoulder joint prosthesis may further include three fasteners 40. A bottom of the outer cup body 11 is provided with three connecting holes 114. The connecting holes 114 are counter bore structures. In the embodiment, the connecting holes 114 are counter bore structures, thereby preventing the fastener 40 from protruding out of the first spherical inner cavity 111 and preventing the fastener 40 from interfering with the mounted lining 30. The three fasteners 40 mount the outer cup body 11 at a scapula through the three connecting holes 114, in this way the outer cup 10 may be fixed.

As shown in Figs. 4-7, three included angles are formed between axes of the three connecting holes 114 and a central axis of the outer cup body 11, with different degrees. The three included angles make fastener 40 fix the outer cup body 11 more firmly, thereby achieving reliable connection. The fastener 40 is a screw preferably.

As for a shoulder glenoid in the conventional art, a shoulder glenoid area of the scapula needs osteotomy which may be harmful to sclerotin, may damage surface cortex of the shoulder glenoid area and not provide the needed fixation with the screw with a good fixation condition.

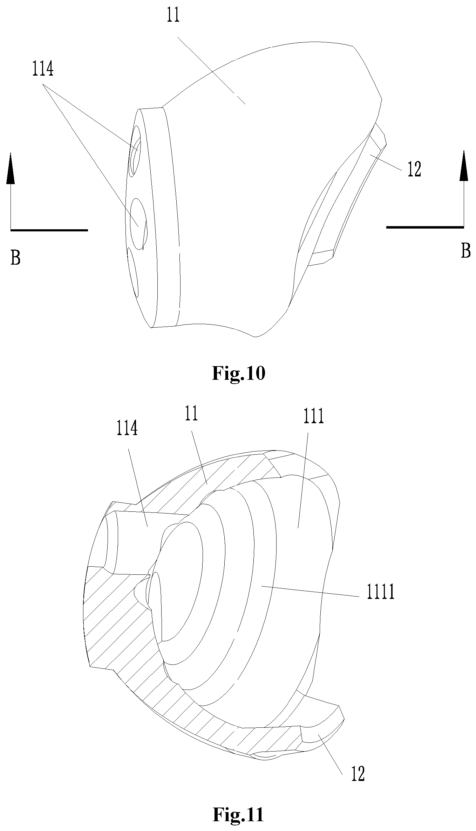

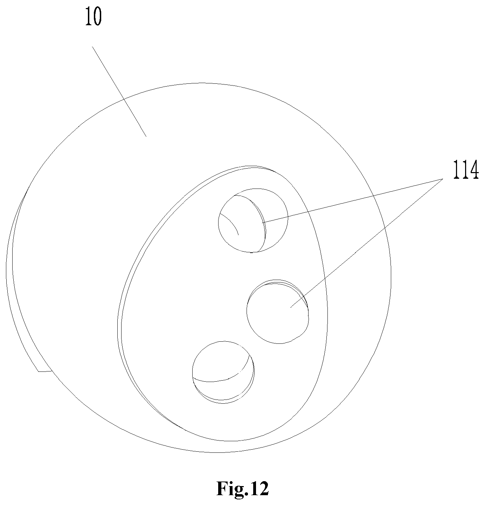

The technical solution of the embodiment may effectively avoid the abovementioned problem. Specifically, as shown in Fig. 10 and Fig. 12, in the embodiment, the outer cup 10 may be prepared from 3D printing titanium alloy, a surface at a bottom of the outer cup 10, contacting with the scapula, may have a matched shape which is formed by means of a 3D printing technology after processing an anatomical feature through a computer graphic technology in accordance with the anatomical feature at a broken position of the scapula of the patient. Therefore, the abovementioned method may achieve individual customization without osteotomy, to accordingly provide the subsequent fixation with the screw with the good fixation condition.

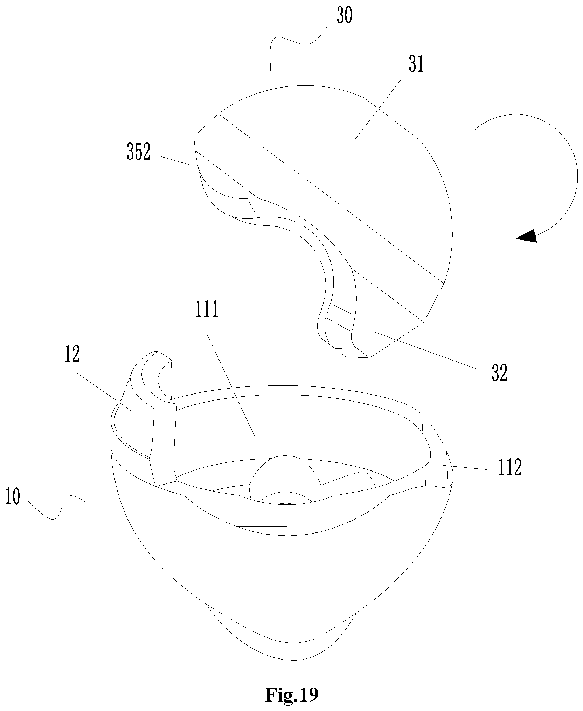

In the embodiment, the ball head 20 is mounted into the second spherical inner cavity 34 of the lining 30 first when assembling the outer cup 10, the lining 30 and the ball head 20, and the lining 30 and the ball head 20 when assembled should be mounted inside the first spherical inner cavity 111 in the rotational motion. In order to facilitate showing a process of mounting the lining 30 inside the outer cup 10 in Fig. 19, the ball head 20 is not shown in Fig. 19. Specifically, the second limiting convex wall 32 is aligned to the first section structure 112 first, to make the second limiting convex wall 32 enter into the first spherical inner cavity 111. A direction of an arrow in Fig. 19 is the one of the lining 30 and the ball head 20 entering into the first spherical inner cavity 111. When an outer wall surface of the hemispherical housing body 31 rotates for a certain angle along the first spherical inner cavity 111, so that an outer wall surface of the second limiting convex wall 32 may fit an inner wall surface of the first limiting convex wall 12, the second groove 352 may be positioned at a position corresponding to the first section structure 112, and the outer wall surface of the hemispherical housing body 31 may completely fit the first spherical inner cavity 111 together. At the moment, the ball head 20 may be assembled inside the first spherical inner cavity 111 of the outer cup 10 jointly with the lining 30. As for the embodiment having the clamping rib 36 and the clamping slot 1112, when the second limiting convex wall 32 corresponds to the inner wall surface of of the first limiting convex wall 12, the clamping rib 36 may be clamped into the clamping slot 1112.

Specifically, the shoulder joint prosthesis may include a shoulder glenoid prosthesis and a humeral prosthesis. The shoulder glenoid prosthesis may include a structure connecting the outer cup 10 with the lining 30 together. Wherein, the outer cup 10 is made of metal, and the lining 30 is a spherical structure having a size of greater than a radius. The ball head 20 is a humeral ball head, and the lining 30 accommodates the humeral ball head into the first spherical inner cavity 111.

In the descriptions of the disclosure, it is to be understood that orientation or position relationships indicated by nouns of locality, such as "front", "back", "upper", "lower", "left", "right", "transverse", "longitudinal", "vertical", "horizontal", "top", "bottom" are generally orientation or position relationships shown in the drawings, are adopted not to indicate or imply that indicated devices or components must be in specific orientations or structured and operated in specific orientations but only to conveniently describe the disclosure and simplify descriptions and thus should not be understood as limits to the disclosure when there is no contrary statement available. The nouns of locality "inside", "outside" are the inside and outside relative to an outline of each part.

In order to facilitate description, a spatial relative term may be used here, such as "over", "above", "on an upper surface" and "on", to describe a spatial position relationship between a device or a feature shown in the figure and other devices or other features. It is to be understood that the spatial relative term is intended to include different orientations of the device during use or operation outside the orientation described in the figure. For example, if the device in the figure is inverted, it may be described as that the device "above other devices or other structures" or "over other devices or other structures" shall be positioned "below other devices or other structures" or "under other devices or other structures". Therefore, an exemplary term "above" may include two orientations: "above" and "below". As an alternative, the device may be positioned with other different modes as well (90° rotation or positioned at other orientations), and the spatial relative description used here needs to be explained correspondingly.

In addition, it is to be noted that the terms "first", "second" and the like are adopted to limit the parts and to facilitate distinguishing corresponding parts only. Unless otherwise stated, the abovementioned terms do not have any special meaning and accordingly should not be understood as the limits to the scope of protection of the disclosure.

## Claims

1. A shoulder joint prosthesis, comprising an outer cup (10), a lining (30), a ball head (20) and a humeral shank (21), the outer cup (10), the lining (30) and the ball head (20) being nested in sequence, wherein,
the lining (30) comprising a hemispherical housing body (31), wherein the lining (30) further comprises a second limiting convex wall (32) and a third limiting convex wall (33) provided at an opening of the hemispherical housing body (31), the second limiting convex wall (32) and the third limiting convex wall (33) protruding out of a center surface of the hemispherical housing body (31), the center surface of the hemispherical housing body (31) being a plane passing a spherical center of the hemispherical housing body (31) and vertical to an axis of the lining (30), the hemispherical housing body (31) being provided with a second spherical inner cavity (34) for accommodating the ball head (20), the second limiting convex wall (32) and the third limiting convex wall (33) limiting the ball head (20) to leave the second spherical inner cavity (34), the hemispherical housing body (31) being provided with a groove (35) for avoiding the humeral shank (21), the groove (35) being lower than the center surface of the hemispherical housing body (31), there are two third limiting convex walls (33) provided oppositely, the second limiting convex wall (32) is positioned between the two third limiting convex walls (33), there are a plurality of grooves (35), the plurality of grooves (35) comprise two first grooves (351) between the two third limiting convex walls (33) and the second limiting convex wall (32) and a second groove (352) between the two third limiting convex walls (33),
wherein the outer cup (10) consists of an outer cup body (11) and one first limiting convex wall (12) provided at an opening of the outer cup body (11), the outer cup body (11) is provided with a first spherical inner cavity (111), the first limiting convex wall (12) protrudes out of a center surface of the first spherical inner cavity (111), the center surface of the first spherical inner cavity (111) is a plane passing a spherical center of the first spherical inner cavity (111) and vertical to an axis of the outer cup (10), and an end surface of the outer cup body (11) is lower than the center surface of the first spherical inner cavity (111); and the lining (30) being configured to be mounted inside the first spherical inner cavity (111) in a rotational motion, wherein, the first limiting convex wall (12) is positioned outside the second limiting convex wall (32), as to limit the lining (30) to leave the first spherical inner cavity (111);
a position on an end surface of the outer cup body (11), corresponding to the second groove (352), is provided with a first section structure (112) for avoiding the humeral shank (21);
a position on an end surface of the outer cup body (11), corresponding to one of the two first grooves (351), is provided with a second section structure (113) for avoiding the humeral shank (21);
**characterized in that** a cavity wall of the first spherical inner cavity (111) is further provided with a clamping slot (1112), the clamping slot (1112) extends along a peripheral direction of the cavity wall of the first spherical inner cavity (111), and an outer wall surface of the lining (30) is provided with a clamping rib (36) corresponding to the clamping slot (1112),
wherein the clamping rib (36) includes a slope portion and a plane portion connecting with the slope portion, the slope portion is provided obliquely relative to the axis of the lining (30), and the plane portion is vertical to the axis of the lining (30).

2. The shoulder joint prosthesis as claimed in claim 1, wherein, a protrusion height of the second limiting convex wall (32) is greater than a protrusion height of the third limiting convex wall (33).

3. The shoulder joint prosthesis as claimed in claim 1, wherein, the lining (30) is an axisymmetric structure, and the second groove (352) and the second limiting convex wall (32) are provided oppositely.

4. The shoulder joint prosthesis as claimed in claim 1, wherein, the groove (35) is an arc groove.

5. The shoulder joint prosthesis as claimed in claim 1, wherein, a cavity wall of the first spherical inner cavity (111) is provided with an annular groove (1111), there are a plurality of annular grooves (1111), and the plurality of annular grooves (1111) are provided along an axial direction of the outer cup body (11) at intervals.

6. The shoulder joint prosthesis as claimed in claim 1, wherein, the shoulder joint prosthesis further comprises a plurality of fasteners (40), a bottom of the outer cup body (11) is provided with a plurality of connecting holes (114), and the plurality of connecting holes (114) are counter bore structures.

7. The shoulder joint prosthesis as claimed in claim 6, wherein, a plurality of included angles are formed between axes of the plurality of connecting holes (114) and a central axis of the outer cup body (11), degrees of the plurality of included angles are different.

## Patentansprüche

1. Schultergelenkprothese, umfassend eine äußere Schale (10), eine Auskleidung (30), einen Kugelkopf (20) und einen Humerusschaft (21), wobei die äußere Schale (10), die Auskleidung (30) und der Kugelkopf (20) nacheinander verschachtelt sind, wobei
die Auskleidung (30) einen halbkugelförmigen Gehäusekörper (31) umfasst, wobei die Auskleidung (30) ferner eine zweite konvexe Begrenzungswand (32) und eine dritte konvexe Begrenzungswand (33) umfasst, die an einer Öffnung des halbkugelförmigen Gehäusekörpers (31) vorgesehen sind, wobei die zweite konvexe Begrenzungswand (32) und die dritte konvexe Begrenzungswand (33) aus einer Mittelfläche des halbkugelförmigen Gehäusekörpers (31) herausragen, wobei die Mittelfläche des halbkugelförmigen Gehäusekörpers (31) eine Ebene ist, die einen kugelförmigen Mittelpunkt des halbkugelförmigen Gehäusekörpers (31) passiert und senkrecht zu einer Achse der Auskleidung (30) verläuft, wobei der halbkugelförmige Gehäusekörper (31) mit einem zweiten kugelförmigen Innenhohlraum (34) zur Unterbringung des Kugelkopfes (20) versehen ist, wobei die zweite konvexe Begrenzungswand (32) und die dritte konvexe Begrenzungswand (33) den Kugelkopf (20) daran begrenzen, den zweiten kugelförmigen Innenhohlraum (34) zu verlassen, wobei der halbkugelförmige Gehäusekörper (31) mit einer Nut (35) zur Vermeidung des Humerusschafts (21) versehen ist, wobei die Nut (35) tiefer gelegen ist als die Mittelfläche des halbkugelförmigen Gehäusekörpers (31), es zwei dritte konvexe Begrenzungswände (33) gibt, die gegenüberliegend vorgesehen sind, die zweite konvexe Begrenzungswand (32) zwischen den zwei dritten konvexen Begrenzungswänden (33) positioniert ist, es eine Vielzahl von Nuten (35) gibt, die Vielzahl von Nuten (35) zwei erste Nuten (351) zwischen den zwei dritten konvexen Begrenzungswänden (33) und der zweiten konvexen Begrenzungswand (32) und eine zweite Nut (352) zwischen den zwei dritten konvexen Begrenzungswänden (33) umfasst,
wobei die äußere Schale (10) aus einem äußeren Schalenkörper (11) und einer ersten konvexen Begrenzungswand (12) besteht, die an einer Öffnung des äußeren Schalenkörpers (11) vorgesehen ist, der äußere Schalenkörper (11) mit einem ersten kugelförmigen Innenhohlraum (111) versehen ist, die erste konvexe Begrenzungswand (12) aus einer Mittelfläche des ersten kugelförmigen Innenhohlraums (111) herausragt, die Mittelfläche des ersten kugelförmigen Innenhohlraums (111) eine Ebene ist, die einen kugelförmigen Mittelpunkt des ersten kugelförmigen Innenhohlraums (111) passiert und senkrecht zu einer Achse der äußeren Schale (10) verläuft, und ein Endfläche des äußeren Schalenkörpers (11) tiefer gelegen ist als die Mittelfläche des ersten kugelförmigen Innenhohlraums (111); und wobei die Auskleidung (30) ausgelegt ist, um innerhalb des ersten kugelförmigen Innenhohlraums (111) in einer Drehbewegung montiert zu werden, wobei die erste konvexe Begrenzungswand (12) außerhalb der zweiten konvexen Begrenzungswand (32) positioniert ist, um die Auskleidung (30) daran zu begrenzen, den ersten kugelförmigen Innenhohlraums (111) zu verlassen;
eine Position auf einer Endfläche des äußeren Schalenkörpers (11), die der zweiten Nut (352) entspricht, mit einer ersten Sektionsstruktur (112) zur Vermeidung des Humerusschafts (21) versehen ist;
eine Position auf einer Endfläche des äußeren Schalenkörpers (11), die einer der zwei ersten Nuten (351) entspricht, mit einer zweiten Sektionsstruktur (113) zur Vermeidung des Humerusschafts (21) versehen ist;
**dadurch gekennzeichnet, dass** eine Hohlraumwand des ersten kugelförmigen Innenhohlraums (111) ferner mit einem Klemmschlitz (1112) versehen ist, sich der Klemmschlitz (1112) entlang einer Umfangsrichtung der Hohlraumwand des ersten kugelförmigen Innenhohlraums (111) erstreckt und eine Außenwandfläche der Auskleidung (30) mit einer dem Klemmschlitz (1112) entsprechenden Klemmrippe (36) versehen ist, wobei
die Klemmrippe (36) einen Neigungsabschnitt und einen ebenen Abschnitt einschließt, der sich mit dem Neigungsabschnitt verbindet, der Neigungsabschnitt schräg relativ zu der Achse der Auskleidung (30) vorgesehen ist und der ebene Abschnitt senkrecht zu der Achse der Auskleidung (30) verläuft.

2. Schultergelenkprothese nach Anspruch 1, wobei eine Vorsprungshöhe der zweiten konvexen Begrenzungswand (32) größer ist als eine Vorsprungshöhe der dritten konvexen Begrenzungswand (33).

3. Schultergelenkprothese nach Anspruch 1, wobei die Auskleidung (30) eine achsensymmetrische Struktur ist und die zweite Nut (352) und die zweite konvexe Begrenzungswand (32) gegenüberliegend vorgesehen sind.

4. Schultergelenkprothese nach Anspruch 1, wobei die Nut (35) eine Bogennut ist.

5. Schultergelenkprothese nach Anspruch 1, wobei eine Hohlraumwand des ersten kugelförmigen Innenhohlraums (111) mit einer Ringnut (1111) versehen ist, es gibt eine Vielzahl von Ringnuten (1111) und die Vielzahl von Ringnuten (1111) entlang einer axialen Richtung des äußeren Schalenkörpers (11) in Abständen vorgesehen ist.

6. Schultergelenkprothese nach Anspruch 1, wobei die Schultergelenkprothese ferner eine Vielzahl von Befestigungselementen (40) umfasst, ein Boden des äußeren Schalenkörpers (11) mit einer Vielzahl von Verbindungslöchern (114) versehen ist und die Vielzahl von Verbindungslöchern (114) Gegenlochstrukturen sind.

7. Schultergelenkprothese nach Anspruch 6, wobei eine Vielzahl von eingeschlossenen Winkeln zwischen Achsen der Vielzahl von Verbindungslöchern (114) und einer Mittelachse des äußeren Schalenkörpers (11) gebildet ist, Grade der Vielzahl von eingeschlossenen Winkeln unterschiedlich sind.

## Revendications

1. Prothèse d'articulation d'épaule, comprenant une coupelle externe (10), un revêtement (30), une tête sphérique (20) et une tige humérale (21), la coupelle externe (10), le revêtement (30) et la tête sphérique (20) étant emboîtés en séquence, dans laquelle le revêtement (30) comprend un corps de logement hémisphérique (31), dans laquelle le revêtement (30) comprend en outre une deuxième paroi convexe de limitation (32) et une troisième paroi convexe de limitation (33) prévues au niveau d'une ouverture du corps de logement hémisphérique (31), la deuxième paroi convexe de limitation (32) et la troisième paroi convexe de limitation (33) faisant saillie à partir d'une surface centrale du corps de logement hémisphérique (31), la surface centrale du corps de logement hémisphérique (31) étant un plan passant par un centre sphérique du corps de logement hémisphérique (31) et vertical à un axe du revêtement (30), le corps de logement hémisphérique (31) étant pourvu d'une seconde cavité interne sphérique (34) destinée à loger la tête sphérique (20), la deuxième paroi convexe de limitation (32) et la troisième paroi convexe de limitation (33) limitant la tête sphérique (20) pour qu'elle ne quitte pas la seconde cavité interne sphérique (34), le corps de logement hémisphérique (31) étant pourvu d'une rainure (35) pour éviter la tige humérale (21), la rainure (35) étant plus basse que la surface centrale du corps de logement hémisphérique (31), deux troisièmes parois convexes de limitation (33) sont prévues de manière opposée, la deuxième paroi convexe de limitation (32) est positionnée entre les deux troisièmes parois convexes de limitation (33), une pluralité de rainures (35) est présente, la pluralité de rainures (35) comprend deux premières rainures (351) entre les deux troisièmes parois convexes de limitation (33) et la deuxième paroi convexe de limitation (32) et une seconde rainure (352) entre les deux troisièmes parois convexes de limitation (33),
dans laquelle la coupelle externe (10) consiste en un corps de coupelle externe (11) et une première paroi convexe de limitation (12) prévue au niveau d'une ouverture du corps de coupelle externe (11), le corps de coupelle externe (11) est pourvu d'une première cavité interne sphérique (111), la première paroi convexe de limitation (12) fait saillie à partir d'une surface centrale de la première cavité interne sphérique (111), la surface centrale de la première cavité interne sphérique (111) est un plan passant par le centre sphérique de la première cavité interne sphérique (111) et vertical à un axe de la coupelle externe (10), et une surface d'extrémité du corps de coupelle externe (11) est plus basse que la surface centrale de la première cavité interne sphérique (111) ; et le revêtement (30) étant configuré pour être monté à l'intérieur de la première cavité interne sphérique (111) dans un mouvement de rotation, dans laquelle la première paroi convexe de limitation (12) est positionnée à l'extérieur de la deuxième paroi convexe de limitation (32), de manière à limiter le revêtement (30) pour qu'il ne quitte pas la première cavité interne sphérique (111) ;
une position sur une surface d'extrémité du corps de coupelle externe (11), correspondant à la seconde rainure (352), est pourvue d'une première structure de section (112) pour éviter la tige humérale (21) ;
une position sur une surface d'extrémité du corps de coupelle externe (11), correspondant à l'une des deux premières rainures (351), est pourvue d'une seconde structure de section (113) pour éviter la tige humérale (21) ;
**caractérisée en ce qu'**une paroi de cavité de la première cavité interne sphérique (111) est en outre pourvue d'une fente de serrage (1112), la fente de serrage (1112) s'étend le long d'une direction périphérique de la paroi de cavité de la première cavité interne sphérique (111), et une surface de paroi externe du revêtement (30) est pourvue d'une nervure de serrage (36) correspondant à la fente de serrage (1112), dans laquelle
la nervure de serrage (36) inclut une partie de pente et une partie plane reliée à la partie de pente, la partie de pente est prévue obliquement par rapport à l'axe du revêtement (30), et la partie plane est verticale par rapport à l'axe du revêtement (30).

2. Prothèse d'articulation d'épaule selon la revendication 1, dans laquelle la hauteur de saillie de la deuxième paroi convexe de limitation (32) est supérieure à la hauteur de saillie de la troisième paroi convexe de limitation (33).

3. Prothèse d'articulation d'épaule selon la revendication 1, dans laquelle le revêtement (30) est une structure axisymétrique, et la seconde rainure (352) et la deuxième paroi convexe de limitation (32) sont prévues de manière opposée.

4. Prothèse d'articulation d'épaule selon la revendication 1, dans laquelle la rainure (35) est une rainure arquée.

5. Prothèse d'articulation d'épaule selon la revendication 1, dans laquelle une paroi de cavité de la première cavité interne sphérique (111) est pourvue d'une rainure annulaire (1111), une pluralité de rainures annulaires (1111) sont prévues, et la pluralité de rainures annulaires (1111) sont prévues le long d'une direction axiale du corps de coupelle externe (11) à intervalles réguliers.

6. Prothèse d'articulation d'épaule selon la revendication 1, dans laquelle la prothèse d'articulation d'épaule comprend en outre une pluralité d'éléments de fixation (40), un fond du corps de la coupelle externe (11) est pourvu d'une pluralité de trous de raccordement (114), et la pluralité de trous de raccordement (114) sont des structures de contrealésage.

7. Prothèse d'articulation d'épaule selon la revendication 6, dans laquelle une pluralité d'angles inclus sont formés entre les axes de la pluralité de trous de raccordement (114) et un axe central du corps de la coupelle externe (11), les degrés de la pluralité d'angles inclus étant différents.
